# EUROPÄISCHE PATENTSCHRIFT

(11) **EP 1 079 753 B1**
(45) Veröffentlichungstag und Bekanntmachung des Hinweises auf die Patenterteilung: **17.12.2003**
(21) Anmeldenummer: 98919023.6
(22) Anmeldetag: 19.05.1998
(51) Int. Cl.: A61B 17/80, A61F 2/44

(54) **OSTEOSYNTHETISCHES IMPLANTAT MIT EINGELASSENER GELENKVERBINDUNG**
OSTEOSYNTHETIC IMPLANT WITH AN EMBEDDED HINGE JOINT
IMPLANT OSTEOSYNTHETIQUE DOTE D'UN ASSEMBLAGE ARTICULE ENCASTRE

(43) Veröffentlichungstag der Anmeldung: 07.03.2001
(73) Patentinhaber: SYNTHES AG Chur, 7002 Chur (CH)
(72) Erfinder: FRIGG, Robert, CH-2544 Bettlach (CH)
(74) Vertreter: Lusuardi, Werther Giovanni, Dr.
(86) Internationale Anmeldenummer: CH9800208
(87) Internationale Veröffentlichungsnummer: WO99059492

(56) Entgegenhaltungen:
- WO-A-88/03781
- DE-A- 1 949 923
- DE-A- 2 438 669
- US-A- 5 683 465

## Beschreibung

Die Erfindung bezieht sich auf eine Vorrichtung, weiche ein osteosynthetisches lmplantat oder lmplantatteil und mindestens ein mit dem lmplantat oder lmplantatteil verbundenes Schwenkmittel umfasst, gemäss dem Oberbegriff des Patentanspruchs 1.

Winkelstabile Längsträger wie Platten und Stäbe usw. verbreiten sich immer weiter in der Osteosynthese. Vorteile bieten sie vor allem bei der Frakturbehandlung in Gelenksnähe oder bei der Schraubenverankerung in der Wirbelsäule. Werden nur kurze Schrauben verwendet, können diese in einem vorgegebenen Winkel in den Längsträger eingesetzt werden. Müssen jedoch längere Schrauben angewendet werden, kann eine systembedingte Richtungsvorgabe der Schraube problematisch sein. Um diese Problematik zu umgehen, wurden spezielle Kugelgelenke (z.B. im Wirbeisäulenbereich) entwickelt. Aus dem Stand der Technik sind auch speziell gestaltete Bohrungen zur Aufnahme von entsprechend gestalteten Schraubenköpfen bekannt, wodurch eine gewisse richtungsunabhängige Blockierung der Knochenschraube in der Knochenplatte ermöglicht wird. Eine Knochenplatte mit solchen speziell gestalteten Schraubenlöchem und entsprechend geformten Schraubenköpfen ist aus der DE 195 48 395 bekannt.

Weder Kugelgelenke noch Schraubenkopfformen mit entsprechend gestalteten Bohrungen in den lmplantatteilen haben sich als optimale Lösungen für das oben erwähnte Problem der systembedingten Richtungsvorgabe für Knochenschrauben erwiesen. Entweder sind die Konstruktionen zu komplex, oder die Belastbarkeit der Verbindung ist ungenügend. Je nach lndikationsort sind diese bekannten Konstruktionen auch zu voluminös.

ln der DE 24 38 669 BEZOLD wird eine Osteosyntheseplatte mit Schraubenlöchern, deren Abstand durch äussere Krafteinwirkung veränderbar ist, offenbart. Die Schraubenlöcher sind in Augen angeordnet, welche vom Hauptteil der Osteosyntheseplatte ausgeschnitten und lediglich durch federelastische Stege mit der Osteosyntheseplatte verbunden sind. ln einer Ausführungsform dieser bekannten Vorrichtung sind die Stege zu einer Achse fluchtend und diametral bezüglich dieser Achse mit dem Auge verbunden. Nachteilig an dieser bekannten Vorrichtung ist, dass beim Schwenken des Auges aus der Plattenebene heraus, die Stege so gebogen werden, dass die Drehachse des Auges nicht mit der Achse des Auges respektive des Schraubenloches zusammenfällt. Daher verschiebt sich beim Drehen des Auges auch das Zentrum des Schraubenloches, was in der klinischen Anwendung negativ ist.

Hier will die Erfindung Abhilfe schaffen. Der Erfindung liegt die Aufgabe zugrunde, eine flache schwenkbare Verbindung zu schaffen, die es ermöglicht, ein lmplantatteil z.B. eine Knochenschraube einfach oder kardanisch schwenkbar mit einem weiteren lmplantatteil z.B. einer Knochenplatte zu verbinden:

Die Erfindung löst die gestellte Aufgabe mit einer Vorrichtung, bestehend aus einem osteosynthetisches lmplantat oder lmplantatteil und mindestens einem mit dem lmplantat oder lmplantatteil verbundenen Schwenkmittel, welche die Merkmale des Anspruchs 1 aufweist.

Weitere vorteilhafte Ausgestaltungen der Erfindung sind in den abhängigen Ansprüchen gekennzeichnet.

ln einer bevorzugten Ausführungsform der erfindungsgemässen Vorrichtung umfasst das innerhalb eines osteosynthetischen Implantates liegende Schwenkmittel zwei flache stabförmige Verbindungsstege; welche einander in einer Achse gegenüberliegen und mit den aussenliegenden Seiten am lmplantat oder lmplantatteil und mit den innenliegenden Seiten an einem platten- oder ringförmiges Schwenkelement angebracht sind. Das Schwenkelement ist über die Verbindungsstege um die durch die Verbindungsstege gebildete Drehachse schwenkbar in einer Bohrung des lmplantates oder lmplantatteils angebracht.

Eine weitere Ausführungsform der erfindungsgemässen Vorrichtung unterscheidet sich von der oben beschriebenen Ausführungsform nur darin, dass lmplantat oder lmplantatteil und die aus Verbindungsstegen und Schwenkelement bestehende Schwenkmittel einteilig sind.

Wiederum eine weitere Ausführungsform der erfindungsgemässen Vorrichtung unterscheidet sich von der oben beschriebenen Ausführungsform nur darin, dass die beiden flachen stabförmigen Verbindungsmittel so dimensioniert sind, dass sie mittels plastischer Verformung eine winkelstabile gegenüber dem Implantat oder lmplantatteil verdrehte Position des Schwenkelementes zulassen.

Eine andere Ausführungsform der erfindungsgemässen Vorrichtung unterscheidet sich von der oben beschriebenen Ausführungsform nur darin, dass anstelle der Verbindungsstege zwischen dem platten- oder ringförmigen Schwenkelement und dem lmplantat oder lmplantatteil Schwenkachsen so angebracht sind, dass das Schwenkelement konzentrisch in einer Bohrung des Implantates oder lmplantatteils gelagert und um die Schwenkachsen drehbar ist.

Wiederum eine andere Ausführungsform der erfindungsgemässen Vorrichtung unterscheidet sich von den vorangehend beschriebenen Ausführungsformen nur dadurch, dass das Schwenkelement eine Dicke a aufweist, die geringer als die Dicke b des lmplantates oder der die Schwenkmittel aufnehmenden Wand des Implantates ist.

Wiederum eine weitere Ausführungsform der erfindungsgemässen Vorrichtung unterscheidet sich von den vorangehend beschriebenen Ausführungsformen nur darin, dass zwei Schwenkelemente so ineinander angeordnet sind, dass das äussere Schwenkelement in der Bohrung im lmplantat oder lmplantatteil angebracht ist und innerhalb einer Bohrung im äusseren Schwenkelement ein weiteres inneres Schwenkelement drehbar gelagert ist.

Wiederum eine weitere Ausführungsform der erfindungsgemässen Vorrichtung unterscheidet sich von den vorangehend beschriebenen Ausführungsformen nur darin, dass bei zwei ineinanderliegenden Schwenkelementen deren durch die stabförmigen Verbindungsmittel gegebenen Drehachsen um 90° versetzt sind.

Wiederum eine andere Ausführungsform der erfindungsgemässen Vorrichtung unterscheidet sich von den vorangehend beschriebenen Ausführungsformen nur dadurch, dass die Schwenkelemente bis auf die Verbindungsstege durch Schlitze vom lmplantat oder lmplantatteil getrennt sind und diese Schlitze kreisringförmig sind.

Eine weitere Ausführungsform der erfindungsgemässen Vorrichtung zeichnet sich dadurch aus, dass die Schwenkmittel in eine Knochenplatte integriert ist.

Wiederum eine weitere Ausführungsform der erfindungsgemässen Vorrichtung zeichnet sich dadurch aus, dass die Schwenkmittel in einen Aufnahmekopf zur Verbindung einer Pedikelschraube mit einem Längsträger innerhalb eines Wirbelsäulenfixationssystems integriert ist.

Wiederum eine andere Ausführungsform der erfindungsgemässen Vorrichtung zeichnet sich dadurch aus, dass je ein Schwenkmittel in der Oberseite und in der Unterseite eines Wirbelsäulenkörpers integriert ist.

Der Wirbelsäulenkörper kann in einer weiteren Ausführungsform so gestaltet sein, dass das die beiden Wirbelkörper-Endplatten verbindende Element ein Stab ist, welcher an seinen beiden Enden senkrecht zu den Drehachsen der erfindungsgemässen Schwenkmittel mit den inneren Schwenkelementen verbunden ist. Die an die Wirbelkörper angrenzenden Wirbelkörper-Endplatten sind als ovale Ringe ausgebildet und mit den Verbindungsstegen der äusseren Schwenkelemente verbunden. Die beiden Wirbelsäulen-Endplatten sind somit je durch ein Schwenkmittel um je mindestens eine Drehachse schwenkbar mit dem Stab verbunden.

Eine weitere Ausführungsform der erfindungsgemässen Vorrichtung unterscheidet sich von den vorangehend beschriebenen Ausführungsformen darin, dass das Schwenkelement mit einer Bohrung zur Aufnahme eines Knochenfixationsmittels, speziell mit einer Knochenschraube oder einer Pedikelschraube versehen ist.

Wiederum eine weitere Ausführungsform der erfindungsgemässen Vorrichtung unterscheidet sich von der vorangehend beschriebenen Ausführungsform dadurch, dass die Bohrung im Schwenkelement zur Aufnahme eines Knochenfixationsmittels konisch ausgebildet ist und das Knochenfixationsmittel bezw. die Knochenschraube oder die Pedikelschraube einen Schraubenkopf mit einem entsprechenden Konus aufweist und damit eine winkelstabile Verbindung zwischen Implantat oder lmplantatteil und Knochenfixationsmittel herstellbar ist.

Eine weitere Ausführungsform der erfindungsgemässen Vorrichtung zeichnet sich gegenüber der vorangehend beschriebenen Ausführungsform dadurch aus, dass die Bohrung im Schwenkelement ein Innengewinde aufweist und das Knochenfixationsmittel bezw. die Knochenschraube oder die Pedikelschraube am Schraubenkopf mit einem entsprechenden Aussengewinde versehen ist und damit eine winkelstabile Verbindung zwischen lmplantat oder lmplantatteil und Knochenfixationsmittel herstellbar ist.

Eine andere Ausführungsform der erfindungsgemässen Vorrichtung zeichnet sich gegenüber der vorangehend beschriebenen Ausführungsform dadurch aus, dass das Knochenfixationsmittel bezw. die Knochenschraube oder die Pedikelschraube mit einem spreizbaren Kopf versehen ist und mittels einer Klemmschraube die Teile dieses spreizbaren Kopfes passgenau gegen die Wandung der Bohrung gepresst werden und damit eine winkelstabile Verbindung zwischen lmplantat oder lmplantatteil und Knochenfixationsmittel herstellbar ist.

Eine weitere Ausführungsform der erfindungsgemässen Vorrichtung zeichnet sich gegenüber der vorangehend beschriebenen Ausführungsform dadurch aus, dass die Bohrung im Schwenkelement ein konisches Innengewinde aufweist und das Knochenfixationsmittel bezw. die Knochenschraube oder die Pedikelschraube am Schraubenkopf mit einem entsprechenden konisches Aussengewinde versehen ist und damit eine winkelstabile Verbindung zwischen lmplantat oder lmplantatteil und Knochenfixationsmittel herstellbar ist.

Wiederum eine weitere Ausführungsform der erfindungsgemässen Vorrichtung zeichnet sich dadurch aus, dass die Schwenkmittel mit einen Aufnahmekopf zur Verbindung mit einem Längsträger innerhalb eines Wirbelsäulenfixationssystems und einer Pedikelschraube einteilig hergestellt ist.

Die durch die Erfindung erreichten Vorteile sind im wesentlichen darin zu sehen, dass dank der erfindungsgemässen Schwenkmittel eine geringere Bauhöhe und eine einfachere Konstruktion als bei Kugelgelenken erreicht werden kann.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt und eine Aufsicht auf eine Knochenplatte, in welcher eine Ausführungsform der erfindungsgemässen Schwenkmittel integriert ist;
Fig. 2 eine Aufsicht auf eine Knochenplatte, in welcher eine andere Ausführungsform der erfindungsgemässen Schwenkmittel integriert ist;
Fig. 3 einen Längsschnitt durch zwei Femurplatten, in weichen eine weitere Ausführungsform der erfindungsgemässen Schwenkmittel integriert ist, mit einer Hüftschraube;
Fig. 4 Aufriss, Seitenriss und eine Ansicht eines Wirbelsäulenfixationselementes, in welchem eine andere Ausführungsform der erfindungsgemässen Schwenkmittel integriert ist;
Fig. 5 eine perspektivische Ansicht eines Wirbelsäutenkörpers, in welchem eine weitere Ausführungsform der erfindungsgemässen Gelenkverbindung integriert ist;
Fig. 6 einen Schnitt durch eine andere Ausführungsform eines Wirbefsäulenkörpers, in welchem eine weitere Ausführungsform der erfindungsgemässen Schwenkmittel integriert ist;
Fig. 7 eine Ansicht des in Fig. 6 dargestellten Wirbelsäulenkörpers; und
Fig. 8 eine Ansicht des in Fig. 6 dargestellten Wirbelsäulenkörpers mit angewinkelten Wirbelkörper-Endplatten.

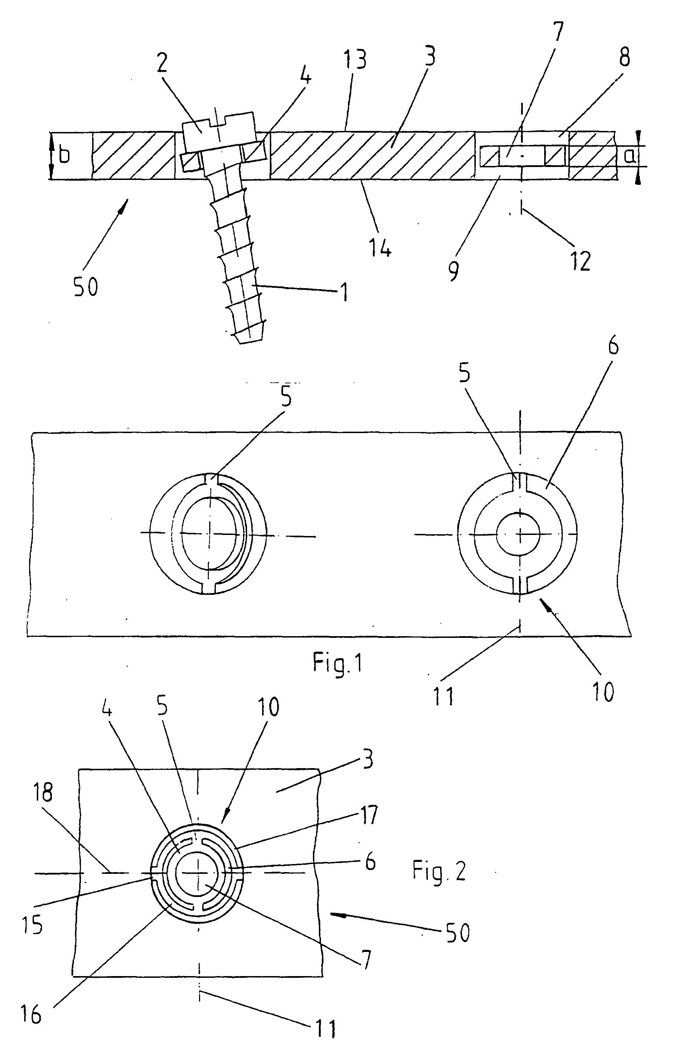

Fig. 1 zeigt eine in einer Knochenplatte 3 angebrachte Ausführungsform der erfindungsgemässen Schwenkmittel 10. Die Knochenplatte 3 ist mit Schraubenlöchem 7, welche von der oberen Fläche 13 zur unteren Fläche 14 der Knochenplatte 3 durchgehen, versehen. Die Schraubenlöcher 7 haben eine Zentralachse 12 und dienen zur Aufnahme von Knochenschrauben 1. Durch Ausfräsen von zwei konzentrisch zu den Schraubenlöchern 7 verlaufenden, aber im Durchmesser grösseren halbkreisförmigen Schlitzen 6 aus der Kochenplatte 3 entsteht das kreisringförmige Schwenkelement 4 mit den beiden einander gegenüberliegenden Verbindungsstegen 5 zur Knochenplatte 3. Verbindungsstege 5 und Schwenkelement 4 sind in der in Fig. 1 dargestellten Ausführungsform mit der Knochenplatte 3 aus einem Stück heraus gefertigt und sind daher Bestandteile derselben. Koaxial zur Zentralachse 12 sind an der Knochenplatte 3 von der oberen Fläche 13 her eine Bohrung 8 und von der unteren Fläche 14 her eine Bohrung 9 so angebracht, dass das Schwenkelement 4 und die Verbindungsstege 5 eine Dicke a aufweisen, die geringer als die Dicke b der Knochenplatte 3 ist. ln der in Fig. 1 gezeigten Ausführungsform der erfindungsgemässen Schwenkmittel 1O besteht diese aus einem einfachen durch die Verbindungsstege 5 gebildeten Gelenk, dessen Drehachse 11 durch die Verbindungsstege 5 und quer zur Längsrichtung der Knochenplatte 3 verläuft. Falls die Knochenschraube 1, deren Schraubenkopf 2 auf dem Schwenkelement 4 aufliegt, schräg in den Knochen geschraubt werden muss, gestatten die erfindungsgemässe Schwenkmittel 10, dass das Schwenkelement 4 vor dem Setzen der Schraube durch einen Stab, der in die Bohrung 7 eingesetzt wird, in die gewünschte Richtung zu den Verbindungselementen 5 geschwenkt wird. Danach kann die Knochenschraube 1 im vorgegebenen Winkel eingeschraubt werden:

Die in Fig. 2 dargestellte Ausführungsform der erfindungsgemässen Schwenkmittel 10 unterscheidet sich von der in Fig. 1 gezeigten Ausführungsform nur dadurch, dass an einem kreisringförmigen äusseren Schwenkelement 16, an welchem innen die Verbindungsstege 5 angebracht sind, aussen weitere Verbindungsstege 15, welche um 90° zu den Verbindungsstegen 5 versetzt sind, angebracht sind. Die äusseren ebenfalls einander gegenüberliegenden Verbindungsstege 15 verbinden das kreisringförmige äussere Schwenkelement 16 mit der Knochenplatte 3 und ermöglichen eine Schwenkbewegung des äusseren Schwenkelementes 16 um eine Achse 18, die parallel zur Längsrichtung der Knochenplatte 3 verläuft. lm lnnern des kreisringförmigen äusseren Schwenkelementes 16 sind die Verbindungsstege 5 angebracht, welche das äussere Schwenkelement 16 und das innere Schwenkelement 4 verbinden und eine Schwenkbewegung des inneren Schwenkelementes 4 um die quer zur Plattenlängsrichtung verlaufende Achse 11 ermöglichen. Die Versetzung der inneren Verbindungsstege 5 um 90° zu den äusseren Verbindungsstegen 15 ermöglicht ein kardanisches Schwenkmittel 1O zwischen dem inneren Schwenkelement 4 und der Knochenplatte 3. Wie bei der in Fig. 1 dargestellten Ausführungsform sind das innere Schwenkelement 4, die Verbindungsstege 5;15 und das äussere Schwenkelement 16 mit der Knochenplatte 3 aus einem Stück heraus gefertigt. Auch bei der hier beschriebenen Ausführungsform wäre eine Ausführung der Verbindungsstege 5;15 durch Stifte mit Lagerungen in der Knochenplatte 3, dem äussern Schwenkelement 16 und dem inneren Schwenkelement 4 möglich.

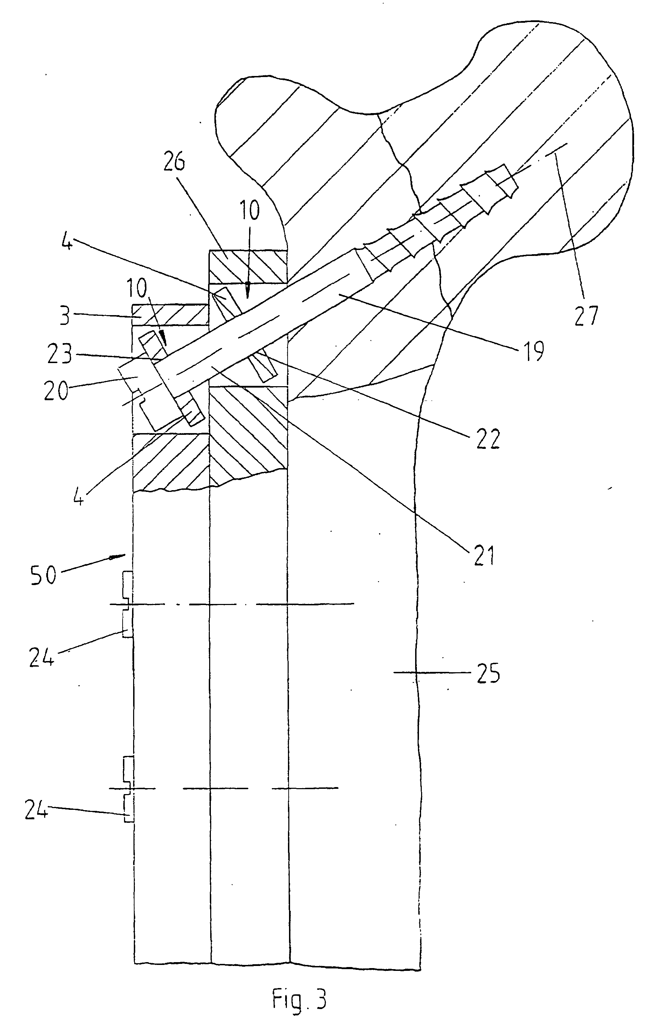

ln Fig. 3 ist eine andere Anwendung der in Fig. 1 oder Fig. 2 dargestellten Knochenplatte 3 mit integrierten Schwenkmitteln 10 dargestellt. Eine Hüftschraube 19, welche die Knochenplatte 3 schräg durchdringt, wird mittels einer der in Fig. 1 oder 2 gezeigten Ausführungsformen der erfindungsgemässen Schwenkmittel 10 in der Knochenplatte 3 gelagert. Genau wie in der in Fig. 1 dargestellten Ausführungsform der Schwenkmittel 10 liegt der Schraubenkopf 20 der Hüftschraube 19 auf dem inneren Schwenkelement 4 auf. Da die Hüftschraube 19 nicht senkrecht zur Knochenplatte 3 verläuft, wird das innere Schwenkelement 4 vor dem Setzen der Hüftschraube 19 in die gewünschte Richtung geschwenkt. Die Schwenkmittel 10 sind wie in Fig. 1 oder 2 dargestellt durch Verbindungsstege 5;15 realisiert. Da die Verbindungsstege 5;15 feste Verbindungen zwischen Knochenplatte 3 und innerem Schwenkelement 4 oder zwischen Knochenplatte 3, äusserem Schwenkelement 16 und innerem Schwenkelement 4 bilden, entsteht beim Ausschwenken der Schwenkmittel 10 eine Rückstellkraft. Damit die Hüftschraube 19 jedoch nur mit axialen Kräften beaufschlagt wird, müssen die erwähnten Rückstellkräfte kompensiert werden. Dies geschieht in der hier dargestellten Anwendung dadurch, dass zwei Knochenplatten 3;26 übereinandergelegt werden und der Schaft 21 der Hüftschraube 19 durch je ein erfindungsgemässes Schwenkmittel 1O durchgeführt wird. Die beiden Knochenplatten 3;26 können mit weiteren Knochenschrauben 24 am Knochen 25 fixiert werden. Die Richtung der Hüftschraube 19 kann durch Verschieben der beiden Knochenplatten 3;26 relativ zueinander eingestellt und festgelegt werden. Durch Anziehen der Knochenschrauben 24 werden die Knochenplatten 3;26 und damit auch die Richtung der Hüftschraube 19 fixiert. Der Schaft 21 der Hüftschraube 19 wird durch die Bohrungen 22;23 in den inneren Schwenkelementen 4 der erfindungsgemässen Schwenkmittel 10 der beiden Knochenplatten 3;26 geführt, so dass bei fixierten Knochenplatten 3;26 trotz der Rückstellkräfte der in den Fig. 1 und 2 gezeigten Verbindungsstege 5;15 keine Kräfte quer zur Längsachse 27 der Hüftschraube 19 auftreten.

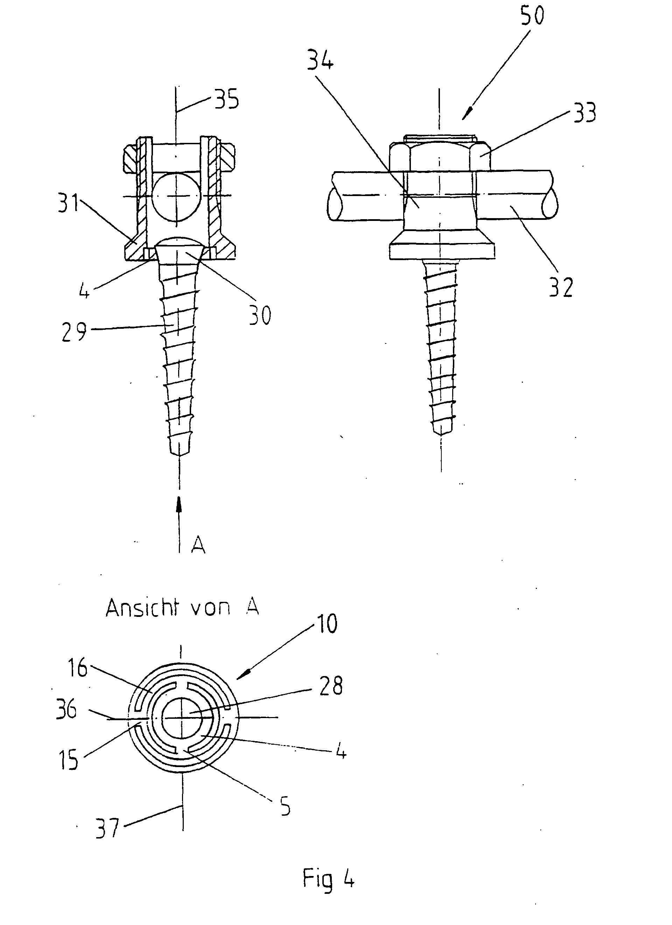

ln Fig. 4 ist eine weitere Anwendung der erfindungsgemässen Schwenkmittel 10 dargestellt. Wie in Fig. 2 gezeigt bestehen die Schwenkmittel 10 aus einem inneren Schwenkelement 4, einem äusseren Schwenkelement 16 und Verbindungsstegen 5;15. Die erfindungsgemässen Schwenkmittel 10 dienen in der hier gezeigten Anwendung zur Verbindung einer Pedikelschraube 29 mit einer Vorrichtung 34 zur Fixierung der Pedikelschraube 29 an einem Längsträger 32 innerhalb eines Wirbefsäulenfixationssystems. Das innere Schwenkelement 4, das äussere Schwenkelement 16 und die inneren Verbindungsstege 5 zwischen innerem Schwenkelement 4 und äusserem Schwenkelement 16 sowie die äusseren Verbindungsstege 16 zwischen dem äusseren Schwenkelement und dem Aufnahmekopf 31 für den Längsträger 31 sind mit dem Aufnahmekopf 31 aus einem Stück gefertigt. Die Pedikelschraube 29 wird durch die dem Schraubenkopf 30 entsprechende Bohrung 28 im inneren Schwenkelement 4 geführt bis der Schraubenkopf 30 in der Bohrung 28 aufliegt. Verläuft die Pedikelschraube 29 nicht parallel zur Längsachse 35 des Aufnahmekopfes 31 wird die Winkeländerung durch die erfindungsgemässe Schwenkmittel 10 aufgenommen. Werden wie in Fig. 2 dargestellt kardanische Schwenkmittel 10 verwendet, kann die Pedikelschraube 29 gegenüber dem Aufnahmekopf 31 um die Achsen 36;37, welche durch die Verbindungsstege 5;15 realisiert werden, geschwenkt werden. Werden wie in Fig. 1 gezeigt einfache Schwenkmittel 10 verwendet, kann die Pedikelschraube 29 nur um eine Achse 36 oder 37 gegenüber dem Aufnahemkopf 31 geschwenkt werden.

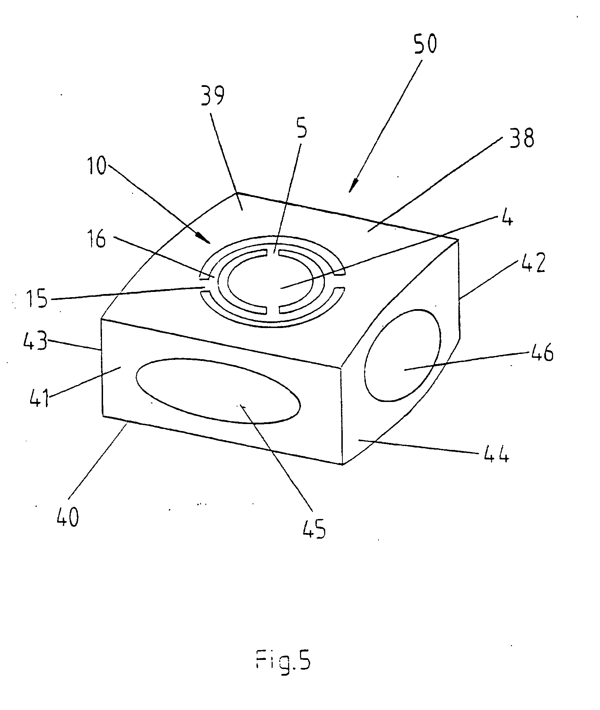

Die in Fig. 5 gezeigte Anwendung einer Ausführungsform der erfindungsgemässen Schwenkmittel 10 umfasst einen Wirbelsäulenkörper 38 und zwei der erfindungsgemässen Schwenkmittel 10, deren innere Schwenkelemente 4 als Auflageflächen für die an den implantierten Wirbelsäulenkörper 38 angrenzenden Wirbelsäulenteile dienen. Der Wirbelsäulenkörper 38 ist als Quader ausgebildet und umfasst eine Oberseite 39, eine Unterseite 40, Vorderseite 41 und Hinterseite 42 sowie zwei Seitenflächen 43 und 44. lm Wirbelsäulenkörper 38 sind eine Durchgangsbohrung 45, welche von der Vorderseite 41 zur Hinterseite 42 verläuft, und eine Durchgangsbohrung 46, weiche zwischen den Seitenflächen 43 und 44 verläuft, angebracht. Oberseite 39 und Unterseite 40 des Wirbelsäulenkörpers 38 sind so gewölbt, dass die inneren Schwenkelemente 4 der auf der Oberseite 39 und auf der Unterseite 40 angebrachten Schwenkmittel 10 die höchstliegende Fläche auf der Oberseite 39 und die tiefstliegende Fläche auf der Unterseite 40 bilden. Damit. ermöglichen die erfindungsgemässen Schwenkmittel 10 bei dieser speziellen Anwendung, dass der Wirbelsäulenkörper 38 auch eingesetzt werden kann, wenn die untere und die obere Auflagefläche in der Wirbelsäule nicht parallel zueinander sind. Die erfindungsgemässen Schwenkmittel 10 sind auf der Oberseite 39 und auf der Unterseite 40 in den Wirbelsäulenkörper 38 integriert, d.h. der Wirbelsäulenkörper 38 und die erfindungsgemässen Schwenkmittel 10 bestehen aus einem Teil. Wie in Fig. 2 gezeigt, bestehen die Schwenkmittel aus einem inneren Schwenkelement 4, welches mittels zwei einander in einer Achse gegenüberliegenden Verbindungsstegen 5 schwenkbar mit einem äusseren Schwenkelement 16 verbunden ist. Das äussere Schwenkelement 16 selbst ist mit zwei einander in einer Achse gegenüberliegenden und gegenüber den inneren Verbindungsstegen 5 um 90° versetzten äusseren Verbindungsstegen 15 schwenkbar mit dem Wirbelsäulenkörper 38 verbunden. Damit ergibt sich für das innere Schwenkelement 4, welches als Auflagefläche für die den Wirbelsäulenkörper 38 berührenden Teile der Wirbelsäule dient, eine Schwenkbarkeit um zwei senkrecht zueinander stehende in der Ebene der Oberseite 39 bzw. der Unterseite 40 des Wirbelsäulenköpers 38 liegende Achsen. Der Wirbelsäulenkörper 38 umfasst in der hier dargestallten Variante je erfindungsgemässes Schwenkmittel 10 auf der Oberseite 39 und auf der Unterseite 40 und kann als Zwischenwirbelelement oder als Wirbelkörperersatz eingesetzt werden.

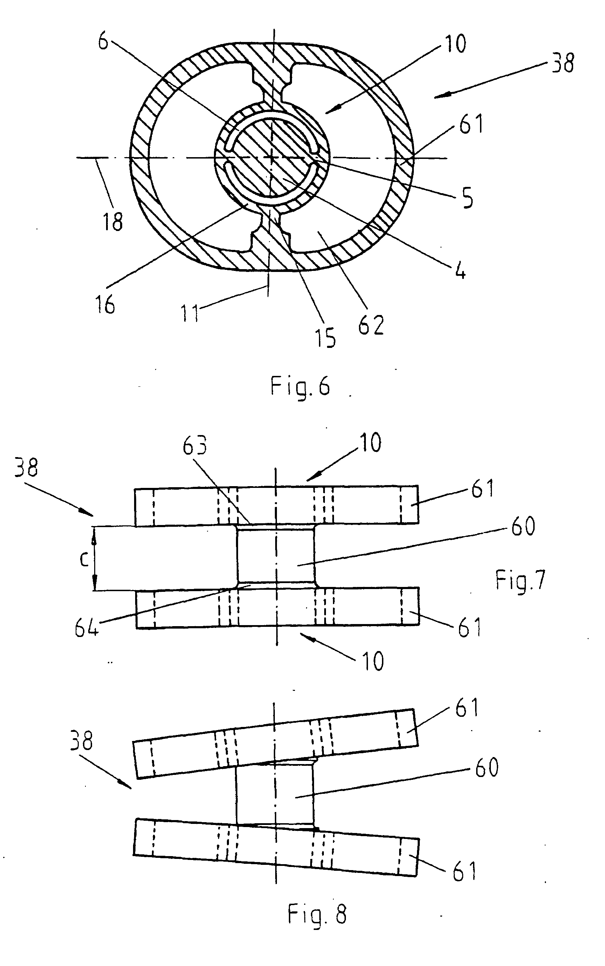

ln Fig. 6, 7 und 8 ist eine andere Ausführungsform des Wirbelsäulenkörpers 38 dargestellt. Die in die Wirbelkörper Endplatten 61 integrierten Schwenkmittel 1O bestehen aus zwei ineinander liegenden, durch Verbindungsstege 5;15 schwenkbar miteinander verbundenen Schwenkelementen 4;16. Beim ungedrehten Schwenkmittel 10 liegen die Schwenkelemente 4;16 in einer durch die Drehachsen 11;18 gebildeten Ebene, die parallel zur Auflagefläche der Wirbelkörper-Endplatten 61 verläuft. Die äusseren ringförmigen Schwenkelemente 16 sind gegen aussen mittels zwei schwenkbaren Verbindungstegen 15 mit den Wirbelkörper Endplatten 61, welche die Form ovaler Ringe aufweisen, drehbar verbunden. Gegen innen sind die äusseren ringförmigen Schwenkelemente 16 ebenfalls mittels Verbindungsstegen 5 mit den inneren Schwenkelementen 4 drehbar verbunden. Die inneren Schwenkelemente 4 sind an den beiden Enden 63;64 eines Stabes (60) angebracht, wobei der Stab 60 jeweils senkrecht auf der durch die Drehachsen 11;18 gebildeten Ebene und somit senkrecht auf dem inneren Schwenkelement 4 steht. Die beiden Wirbelsäulen-Endplatten 61 sind somit je durch ein Schwenkmittel 10 um je zwei Drehachsen 11;18 schwenkbar mit dem Stab 60 verbunden.

## Patentansprüche

1. Vorrichtung, welche ein osteosynthetisches lmplantat oder lmplantatteil und mindestens ein mit dem lmplantat oder lmplantatteil verbundenes Schwenkmittel (10) umfasst,
**dadurch gekennzeichnet, dass**
A) das mindestens eine Schwenkmittel (10) als flaches Schwenkgelenk ausgebildet ist;
B) das mindestens eine Schwenkmittel (10) mindestens ein flaches platten- oder ringförmiges Schwenkelement (4;16) und pro Schwenkelement (4;16) zwei flache stabförmige Verbindungsmittel (5;15), welche in einer Achse (11;18;36;37) liegend am äusseren Umfang des Schwenkelementes (4;16) angebracht sind und die Drehachsen bilden, umfasst; und
C) pro Schwenkmittel (10) eines der Schwenkelemente (4;16) mittels der Verbindungsmittel (5;15) mit dem Implantat oder lmplantatteil schwenkbar verbunden ist.

2. Vorrichtung nach Anspruch 1, **dadurch gekennzeichnet, dass** das innere Schwenkelement (4) mit einer senkrecht zu dessen durch die Schwenkachsen (11;18;36;37) definierten Schwenkebene verlaufenden Bohrung (7;22;23;28) versehen ist.

3. Vorrichtung nach Anspruch 1 oder 2, **dadurch gekennzeichnet, dass** Implantat oder lmplantatteil einerseits und Schwenkmittel (10) andererseits einteilig ausgebildet sind.

4. Vorrichtung nach einem der Ansprüche 1 bis 3, **dadurch gekennzeichnet, dass** die beiden flachen stabförmigen Verbindungsmittel (5;15) so dimensioniert sind, dass mittels plastischer Deformation der beiden Verbindungsmittel (5;15) eine winkelstabile, gegenüber dem lmplantat oder lmplantatteil verdrehte Position des Schwenkelementes (4;16) erreichbar ist.

5. Vorrichtung nach einem der Ansprüche 1 bis 4, **dadurch gekennzeichnet, dass** das Schwenkmittel (10) zwei ineinander in einer Ebene angeordnete Schwenkelemente (4;16) umfasst, wobei das jeweils innere Schwenkelement (4) durch zwei Verbindungsmittel (5) um eine erste Achse (11;37) schwenkbar mit dem jeweils äusseren Schwenkelement (16) verbunden ist und das jeweils äussere Schwenkelement (16) durch zwei Verbindungsmittel (15) um eine zweite Achse (18;36) schwenkbar mit dem lmplantat oder lmplantatteil verbunden ist.

6. Vorrichtung nach Anspruch 5, **dadurch gekennzeichnet, dass** die Schwenkachse (11;37) zwischen den ineinander angeordneten Schwenkelementen (4;16) in der Ebene, in der die Schwenkelemente (4;16) liegen, angeordnet ist.

7. Vorrichtung nach Anspruch 5 oder 6, **dadurch gekennzeichnet, dass** die Schwenkachse (11;37) zwischen den ineinander angeordneten Schwenkelementen (4;16) um 90° gegenüber der Schwenkachse (18) zwischen dem äusseren Schwenkelement (16) und dem lmplantat oder Implantatteil versetzt ist.

8. Vorrichtung nach einem der Ansprüche 1 oder 4 bis 6, **dadurch gekennzeichnet, dass** die flachen stabförmigen Verbindungsmittel (5;15) in dem mindestens einen. Schwenkelement (4;16) und im lmplantat oder lmplantatteil (3;26;31;39) drehbar gelagerte Achsen sind.

9. Vorrichtung nach einem der Ansprüche 1 bis 7, **dadurch gekennzeichnet, dass** die Schwenkelemente (4;16) und die lmplantate oder lmplantatteile voneinander durch Schlitze (6;17) bis auf die Verbindungsmittel (5;15) getrennt sind.

10. Vorrichtung nach Anspruch 9, **dadurch gekennzeichnet, dass** die Schlitze (6;17) abschnittsweise kreisringförmig sind.

11. Vorrichtung nach einem der Ansprüche 1 bis 10, **dadurch gekennzeichnet, dass** das lmplantat, mit welchem das Schwenkmittel (10) verbunden ist, eine quaderförmig Knochenplatte (3) mit einer Dicke b ist.

12. Vorrichtung nach Anspruch 11, **dadurch gekennzeichnet, dass** das mindestens eine Schwenkelement (4;15) eine Dicke a aufweist, die geringer als die Dicke b der Knochenplatte (3) ist.

13. Vorrichtung nach Anspruch 11 oder 12, **dadurch gekennzeichnet, dass** die flachen stabförmigen Verbindungsmittel (5;15) eine Dicke a aufweisen, die geringer als die Dicke b der Knochenplatte (3) ist.

14. Vorrichtung nach einem der Ansprüche 1 bis 10, **dadurch gekennzeichnet, dass** das Schwenkmittel (10) in einen Aufnahmekopf (31) zur Verbindung einer Pedikelschraube (29) mit einem Längsträger (32) innerhalb eines Wirbelsäulenfixationssystems integriert ist.

15. Vorrichtung nach Anspruch 14, **dadurch gekennzeichnet, dass** das Schwenkmittel (10) mit mindestens einem Schwenkelement (4), der Aufnahmekopf (31) zur Verbindung mit dem Längsträger (32) und die Pedikelschraube (29) eine einteilige Einheit bilden.

16. Vorrichtung nach einem der Ansprüche 1 bis 10, **dadurch gekennzeichnet, dass** das lmplantat als Zwischenwirbelkörper (38) oder Wirbelersatzkörper ausgebildet ist.

17. Vorrichtung nach Anspruch 16, **dadurch gekennzeichnet, dass** je ein Schwenkmittel (10) mit je einer an die benachbarten Wirbelkörper adaptierbaren Fläche in der Oberseite (39) und in der Unterseite (40) eines Zwischenwirbelkörpers (38) integriert ist.

18. Vorrichtung nach Anspruch 16, **dadurch gekennzeichnet, dass** zwei Schwenkmittel (10) an ihren inneren Schwenkelementen (4) senkrecht zu den Schwenkachsen (11;18) an den Enden (63;64) eines Stabes (60) mit einer Länge c befestigt sind und an jedem Schwenkmittel (10) ein Schwenkelement (4;16) mit einer Wirbelkörper-Endplatte (61) verbunden ist.

19. Vorrichtung nach einem der Ansprüche 1 bis 14, **dadurch gekennzeichnet, dass** das Schwenkmittel (10) zur Aufnahme eines Knochenfixationsmittels ausgebildet ist.

20. Vorrichtung nach Anspruch 19, **dadurch gekennzeichnet, dass** die Vorrichtung als Knochenfixationsmittel zusätzlich eine Knochenschraube (1;19), die durch die Bohrung (7) im Schwenkelement (4) durchgeht und deren Schraubenkopf (2) auf dem Schwenkelement (4) aufliegt, umfasst und diese Knochenschraube (1;19) mittels des Schwenkmittels (10) um mindestens eine Achse (11;18) drehbar in der Knochenplatte (3) aufgenommen wird.

21. Vorrichtung nach Anspruch 19, **dadurch gekennzeichnet, dass** die Vorrichtung als Knochenfixationsmittel zusätzlich eine Pedikelschraube (29), die durch die Bohrung (28) im Schwenkelement (4) durchgeht und deren Schraubenkopf (30) auf dem Schwenkelement (4) aufliegt, umfasst und diese Pedikelschraube (29) mittels des Schwenkmittels (10) um mindestens eine Achse (36;37) drehbar im Aufnahmekopf (31 ) des Wirbelsäulenfixationssystems (50) aufgenommen wird.

22. Vorrichtung nach Anspruch 20 oder 21, **dadurch gekennzeichnet, dass** die Bohrung (28) im Schwenkelement (4) konisch ausgebildet ist und das Knochenfixationsmittel bezw. Die Knochenschraube (1;19) oder die Pedikelschraube (29) einen Schraubenkopf (2;20;30) mit einem entsprechenden Konus aufweist und damit eine winkelstabile Verbindung zwischen lmplantat oder lmplantatteil und Knochenfixationsmittel herstellbar ist.

23. Vorrichtung nach Anspruch 20 oder 21, **dadurch gekennzeichnet, dass** die Bohrung (28) im Schwenkelement (4) einlnnengewinde aufweist und das Knochenfixationsmittel bezw. Die Knochenschraube (1;19) oder die Pedikelschraube (29) am Schraubenkopf (2;20;30) oder an einem Schaftteil passgenau mit einem entsprechenden Aussengewinde versehen ist und damit eine winkelstabile Verbindung zwischen lmplantat oder lmplantatteil und Knochenfixationsmittel herstellbar ist.

24. Vorrichtung nach Anspruch 20 oder 21, **dadurch gekennzeichnet, dass** die Bohrung (28) im Schwenkelement (4) ein konisches lnnengewinde aufweist und das Knochenfixationsmittel bezw. Die Knochenschraube (1;19) oder die Pedikelschraube (29) am Schraubenkopf (2;20;30) oder an einem Schaftteil passgenau mit einem entsprechenden konischen Aussengewinde versehen ist und damit eine winkelstabile Verbindung zwischen Implantat oder lmplantatteil und Knochenfixationsmittel herstellbar ist.

25. Vorrichtung nach Anspruch 20 oder 21, **dadurch gekennzeichnet, dass** das Knochenfixationsmittel bezw. die Knochenschraube (1;19) oder die Pedikelschraube (29) mit einem spreizbaren Kopf (2;20;30) versehen ist und mittels einer Klemmschraube die Teile dieses spreizbare Kopfes passgenau gegen die Wandung der Bohrung (28) gepresst werden und damit eine winkelstabile Verbindung zwischen lmplantat oder lmplantatteil und Knochenfixationsmittel herstellbar ist.

26. Vorrichtung nach einem der Ansprüche 1 bis 25, **dadurch gekennzeichnet, dass** das Schwenkmittel (10) als flaches Kardan-Schwenkmittel ausgebildet ist.

## Claims

1. A device comprising an osteo-synthetic implant or implant element and at least one swivel means (10) connected to the implant or implant element,
**characterized in that**
a) at least one swivel means (10) is in the form of a flat gimbal,
b) the at least one swivel means (10) comprises at least one planar or annular gimbal (4; 16) and two flat, bar-shaped connecting arms (5: 15) per gimbal which are mounted in one axis (11; 18; 36; 37) and at the outer periphery of the gimbal (4; 16) and which form the axes of rotation, and
c) one gimbal (4; 16) per swivel means (10) is pivotably connected by the connecting arms (5: 15) to the implant or implant element

2. Device as claimed in claim 1, **characterized in that** the inner gimbal (4) is fitted with a borehole (7; 22; 23; 28) running perpendicularly to the gimbal's plane of pivoting defined by the pivoting axes (11; 18; 36; 37).

3. Device as claimed in either of claims 1 and 2, **characterized in that** the implant or implant element on one hand and the swivel means (10) on the other hand are integral.

4. Device as claimed in one of claims 1 through 3, **characterized in that** the two flat, bar-shaped connecting arms (5; 15) are dimensioned in such a way that due to the plastic deformation of the two connecting arms (5; 15) an angularly fixed position of the gimbal (4; 16) is achieved relative to the implant or implant element.

5. Device as claimed in one of claims 1 through 4, **characterized in that** the swivel means (10) comprises two gimbals (4; 16) nesting in each other in one plane, the particular inner gimbal (4) being connected by two connecting arms (5) to the particular outer gimbal (16) so as to be pivotable about a first axis (11; 37)and **in that** the particular outer gimbal (16) is connected by two connecting arms (15) to the implant or implant element so as to be pivotable about a second axis (18; 36).

6. Device as claimed in claim 5, **characterized in that** the pivot axis (11; 37) runs between the mutually nested gimbals (4; 16) and in the plane they subtend.

7. Device as claimed in either of claims 5 and 6, **characterized in that** the pivot axis (11; 37) between the mutually nested gimbals (4; 16) is offset by 90ø relative to the pivot axis (18) between the outer gimbal (16) and the implant or implant element.

8. Device as claimed in one of claims 1 or 4 through 6, **characterized in that** the flat, bar-shaped connecting arms (5: 15) in the at least one gimbal (4: 16) and in the implant or implant element (3; 26; 31; 39) are rotatably supported shafts.

9. Device as claimed in one of claims 1 through 7, **characterized in that** the gimbals (4; 16) and the implants or implant elements are mutually separated by slots (6; 17) as far as the connecting arms (5; 15).

10. Device as claimed in claim 9, **characterized in that** the slots (6; 17)are arcuate segments.

11. Device as claimed in one of claims 1 through 10, **characterized in that** the implant to which is affixed the swivel means (10) is an approximately right parallelipipedic bone plate (3) of thickness "b".

12. Device as claimed in claim 11, **characterized in that** the at least one gimbal (4; 15) is of thickness "a" less than the thickness "b" of the bone plate (3).

13. Device as claimed in either of claims 11 and 12, **characterized in that** the flat, bar-shaped connecting arms (5; 15) are of thickness "a" less than the thickness "b" of the bone plate (3).

14. Device as claimed in one of claims 1 through 10, **characterized in that** the swivel means (10) is integrated into a receiving head (31) to connect a pedicle screw (29) to a longitudinal support (32) within a vertebral affixation system.

15. Device as claimed in claim 14, **characterized in that** the swivel means (10) forms an integral unit with at least one gimbal (4), with the receiving head (31) for connection to the longitudinal support (32) and with the pedicle screw (29).

16. Device as claimed in one of claims 1 through 10, **characterized in that** the implant is in the form of an inter-vertebral unit (38) or a vertebral replacement unit.

17. Device as claimed in claim 16, **characterized in that** one swivel means (10) each is integrated in an inter vertebral unit (38) in a surface that can be matched to the adjacent vertebras both at the top side (39) and at the bottom side (40):

18. Device as claimed in claim 16, **characterized in that** two swivel means (10) are affixed by their inner gimbals (4) perpendicularly to the pivot axes (11; 18) to the ends (63; 64) of a bar (60) of length "c" and **in that** a gimbal (4; 16) is connected at each swivel means (10) to a vertebral end-plate (61).

19. Device as claimed in one of claims 1 through 14, **characterized in that** the swivel means (10) is designed to receive a bone affixation means.

20. Device as claimed in claim 19, **characterized in that** the device acting as bone affixation means additionally comprises a bone screw (1; 19) passing through the borehole (7) in the gimbal (4) and of which the screw head (2) rests on the gimbal (4) and **in that** this bone screw (1; 19) is received by means of the swivel means (10) in the bone plate (3) so as to be rotatable about at least one axis (11; 18).

21. Device as claimed in claim 19, **characterized in that** it comprises an additional pedicle screw (29) as the bone affixation means, this screw (29) passing through the borehole (28) in the gimbal (4) and its head (30) resting on the gimbal (4), and **in that** this pedicle screw (29) is received by means of the swivel means (10) in the receiving head (31) of the vertebral affixation system (50) so as to be rotatable about at least one axis (36; 37).

22. Device as claimed in either of claims 20 and 21, **characterized in that** the borehole (28) in the gimbal (4) is conical and **in that** the bone affixation means, ie the bone screw (1; 19) or the pedicle screw (29) comprises a screw head (2; 20; 30) fitted with a corresponding cone and **in that** thereby a fixed angular connection can be established between the implant or implant element and the bone affixation means.

23. Device as claimed in either of claims 20 and 21, **characterized in that** the borehole (28) in the gimbal (4) is fitted with an inside thread and **in that** the bone affixation means or the bone screw (1; 19) or the pedicle screw (29) is snugly provided at the screw head (2; 20; 30) or at a portion of the shank with a corresponding outer thread and **in that** thereby an angularly fixed connection can be established between the implant or implant element and the bone affixation means.

24. Device as claimed in either of claims 20 and 21, **characterized in that** the borehole (28) in the gimbal (4) is fitted with an inside conical thread and **in that** the bone affixation means or the bone screw (1; 19) or the pedicle screw (29) is snugly fitted at the screw head (2; 20; 30) or at a shank segment with a corresponding outside thread and **in that** thereby an angularly fixed connection can be established between the implant or implant element and the bone affixation means.

25. Device as claimed in either of claims 20 and 21, **characterized in that** the bone affixation means or the bone screw (1; 19) or the pedicle screw (29) is fitted with an expansible head (2; 20; 30) and **in that**, by means of a tightening screw, the parts of this expansible head are press-fitted against the wall of the borehole (28) and that thereby an angularly fixed connection can be established between the implant or implant element and the bone affixation means.

26. Device as claimed in one of claims 1 through 25, **characterized in that** the swivel means (10) is a planar gimbal system.

## Revendications

1. Dispositif qui comprend un implant d'ostéosynthèse ou une partie d'implant d'ostéosynthèse et au moins un moyen de pivotement (10) relié à l'implant ou à la partie d'implant,
**caractérisé en ce que**
A) le moyen de pivotement (10), au moins un est configuré comme articulation pivotante plate,
B) le moyen de pivotement (10), au moins un, comprend au moins un élément de pivotement (4; 16) en forme de plaque aplatie ou en forme d'anneau et pour chaque élément de pivotement (4; 16), deux moyens de liaison (5; 15) en forme de barreaux qui sont situés sur un axe (11; 18; 36; 37) à la périphérie extérieure de l'élément de pivotement (4; 16) et qui forment des axes de rotation et
C) pour chaque moyen de pivotement (10) l'un des éléments de pivotement (4; 16) est relié en rotation à l'implant ou à la partie d'implant à l'aide de moyens de liaison (5; 15).

2. Dispositif selon la revendication 1, **caractérisé en ce que** l'élément intérieur de pivotement (4) est doté d'un alésage (7; 22; 23; 28) qui s'étend perpendiculairement à son plan de pivotement qui est défini par les axes de pivotement (11; 18; 36; 37).

3. Dispositif selon la revendication 1 ou 2, **caractérisé en ce que** l'implant ou la partie d'implant, d'une part, et les moyens de pivotement (10), d'autre part, sont réalisés d'une seule pièce.

4. Dispositif selon l'une des revendications 1 à 3, **caractérisé en ce que** les deux moyens de liaison (5; 15) en forme de barreaux plats sont dimensionnés de telle sorte que par déformation plastique des deux moyens de liaison (5; 15) on puisse obtenir une position de l'élément de pivotement (4; 16) décalée d'un angle fixe par rapport à l'implant ou à la partie d'implant.

5. Dispositif selon l'une des revendications 1 à 4, **caractérisé en ce que** le moyen de pivotement (10) comprend deux éléments de pivotement (4; 16) disposés l'un dans l'autre dans un plan, l'élément intérieur de pivotement (4) étant relié à l'élément extérieur de pivotement (16) correspondant à pivotement autour d'un premier axe (11; 37) par deux moyens de liaison (5), l'élément extérieur de pivotement (16) étant relié à l'implant ou à la partie d'implant à pivotement autour d'un deuxième axe (18; 36) par deux moyens de liaison (15).

6. Dispositif selon la revendication 5, **caractérisé en ce que** l'axe de pivotement (11; 37) situé entre les deux éléments de pivotement (4; 16) disposés l'un à l'intérieur de l'autre est disposé dans le plan dans lequel sont situés les éléments de pivotement (4; 16).

7. Dispositif selon les revendications 5 ou 6, **caractérisé en ce que** l'axe de pivotement (11; 37) situé entre les deux éléments de pivotement (4; 16) disposés l'un à l'intérieur de l'autre est décalé de 90° par rapport à l'axe de pivotement (18) entre l'élément extérieur de pivotement (16) et l'implant ou la partie d'implant.

8. Dispositif selon l'une des revendications 1 ou 4 à 6, **caractérisé en ce que** les moyens de liaison (5; 15) en forme de barreaux plats sont des axes montés à rotation dans au moins un élément de pivotement (4; 16) et dans l'implant ou la partie d'implant (3; 26; 31; 39).

9. Dispositif selon l'une des revendications 1 à 7, **caractérisé en ce que** les éléments de pivotement (4; 16) et les implants ou parties d'implants sont séparés les uns des autres par des fentes (6,17) qui s'étendent jusqu'aux moyens de liaison (5,15).

10. Dispositif selon la revendication 9, **caractérisé en ce que** les fentes (6,17) présentent des parties en forme d'anneaux circulaires.

11. Dispositif selon l'une des revendications 1 à 10, **caractérisé en ce que** l'implant auquel le moyen de pivotement (10) est relié est une plaque d'ostéosynthèse (3) d'une épaisseur b.

12. Dispositif selon la revendication 11, **caractérisé en ce que** l'élément de pivotement (4; 15), au moins un, présente une épaisseur a qui est inférieure à l'épaisseur b de la plaque d'ostéosynthèse (3).

13. Dispositif selon la revendication 11 ou 12, **caractérisé en ce que** les moyens de liaison (5; 15) en forme de barreaux plats ont une épaisseur a qui est inférieure à l'épaisseur b de la plaque d'ostéosynthèse (3).

14. Dispositif selon l'une des revendications 1 à 10, **caractérisé en ce que** le moyen de pivotement (10) est intégré dans une tête de réception (31) destinée à relier une vis à pédicule (29) à un support longitudinal 32) dans un système de fixation de la colonne vertébrale.

15. Dispositif selon la revendication 14, **caractérisé en ce que** le moyen de pivotement (10) qui comprend au moins un élément de pivotement (4), la tête de réception (31) qui assure la liaison avec le support longitudinal (32) et la vis à pédicule (29) forment une entité d'une seule pièce.

16. Dispositif selon l'une des revendications 1 à 10, **caractérisé en ce que** l'implant est configuré comme corps intervertébral (38) ou corps de remplacement de vertèbres.

17. Dispositif selon la revendication 16, **caractérisé en ce qu'**un moyen de pivotement (10) est intégré à une surface adaptée au corps de vertèbre voisin dans la face supérieure (39) et dans la face intérieur (40) d'un corps intervertébral (38).

18. Dispositif selon la revendication 16, **caractérisé en ce que** deux moyens de pivotement (10) sont fixés sur leur élément intérieur de pivotement (4) perpendiculairement aux axes de pivotement (11; 18) aux extrémités (63; 64) d'un barreau (60) de longueur c, un élément de pivotement (4; 16) doté d'une plaque d'extrémité (61) de corps de vertèbre étant relié à chaque élément de pivotement (10).

19. Dispositif selon l'une des revendications 1 à 14, **caractérisé en ce que** le moyen de pivotement (10) est configuré pour recevoir un moyen de fixation de l'os.

20. Dispositif selon la revendication 19, **caractérisé en ce que** le dispositif qui sert de moyen de fixation de l'os comprend de plus une vis d'ostéosynthèse (1; 19) qui traverse l'alésage (7) ménagé dans l'élément de pivotement (4) et dont la tête (2) repose sur l'élément de pivotement (4), cette vis d'ostéosynthèse (1; 19) étant reçue dans la plaque d'ostéosynthèse (3) de manière à pouvoir tourner autour d'au moins un axe (11; 18) à l'aide du moyen de pivotement (10).

21. Dispositif selon la revendication (19), **caractérisé en ce que** le dispositif utilisé comme moyen de fixation de l'os comprend de plus une vis à pédicule (29) qui traverse l'alésage (28) ménagé dans l'élément de pivotement (4) et dont la tête (30) repose sur l'élément de pivotement (4), cette vis à pédicule (29) étant reçue dans la tête de réception (31) du système (50) de fixation de la colonne vertébrale de manière à pouvoir tourner autour d'au moins un axe (36,37) à l'aide du moyen de pivotement (10).

22. Dispositif selon la revendication 20 ou 21, **caractérisé en ce que** l'alésage (28) ménagé dans l'élément de pivotement (4) a une forme conique et **en ce que** le moyen de fixation de l'os, la vis d'ostéosynthèse (1; 19) ou la vis à pédicule (29) présentent une tête de vis (2; 20; 30) avec un cône correspondant, ce qui permet d'établir une liaison à angle fixe entre l'implant ou la partie d'implant et le moyen de fixation de l'os.

23. Dispositif selon la revendication 20 ou 21, **caractérisé en ce que** l'alésage (28) ménagé dans l'élément de pivotement (4) présente un filet intérieur et **en ce que** le moyen de fixation de l'os, la vis d'ostéosynthèse (1; 19) ou la vis à pédicule (29) sont dotés sur la tête de vis (2; 20; 30) ou sur une partie de tige d'un filet extérieur correspondant ajusté étroitement, ce qui permet d'établir une liaison sous un angle fixe entre l'implant ou la partie d'implant et le moyen de fixation de l'os.

24. Dispositif selon les revendications 20 ou 21, **caractérisé en ce que** l'alésage (28) ménagé dans l'élément de pivotement (4) présente un filet intérieur conique et **en ce que** le moyen de fixation de l'os,; la vis d'ostéosynthèse (1; 19) ou la vis à pédicule (29) sont dotés sur la tête de vis (2; 20; 30) ou sur une partie de tige de cette vis d'un filet extérieur conique correspondant ajusté étroitement, ce qui permet d'établir une liaison à angle fixe entre l'implant ou la partie d'implant et le moyen de fixation de l'os.

25. Dispositif selon les revendications 20 ou 21, **caractérisé en ce que** les moyens de fixation de l'os, la vis d'ostéosynthèse (1:19) ou la vis à pédicule (29) sont dotés d'une tête expansible (2; 20; 30) et les parties de cette tête expansible peuvent être repoussées de manière étroitement ajustée contre la paroi de l'alésage (28) au moyen d'une vis de serrage, ce qui permet d'établir une liaison à angle fixe entre l'implant ou la partie d'implant et le moyen de fixation de l'os.

26. Dispositif selon l'une des revendications 1 à 25, **caractérisé en ce que** les moyens de pivotement (10) sont configurés comme moyens de pivotement plats à cardan.
